# EUROPEAN PATENT APPLICATION

(11) **EP 1 889 916 A1**
(43) Date of publication of application: **20.02.2008**
(21) Application number: 06722298.4
(22) Date of filing: 11.04.2006
(51) Int. Cl.: C12N 15/63, C12N 15/70, C12N 15/09, C12N 5/00, C07K 14/475

(54) **A METHOD FOR THE PRODUCTION OF THE RECOMBINANT HOXB4 PROTEIN CONTAINING HIS TAG AT C-TERMINAL AND USE THEREOF**

(30) Priority: 10.02.2006 CN 200610003108
(71) Applicant: Taiwan Advance Bio-Pharm Inc., Taiwan (CN)
(72) Inventor: WU, Kou-juey, Taiwan (CN)
(74) Representative: Viering, Jentschura & Partner
(86) International application number: PCT/CN2006/000646
(87) International publication number: WO 2007/090317

(57) **Abstract**

The present invention relates to a process for making the improved C-terminal Histidine tagged HOXB4 recombinant protein (TAT-HOXB4H) which containing 6 Histidine residues in the C-terminus. The generation of the C-terminal Histidine tag increases the amount of TAT-HOXB4H recombinant protein to be purified about 3-4 fold compared to the original TAT-HOXB4. The present invention also relates to a construct for preparing the TAT-HOXB4H recombinant protein with increased yield to facilitate expansion of stem cells.

SEQUENCE LISTING

<110> TAIWAN ADVANCED BIO-PHARM INC.

<120> A METHOD FOR THE PRODUCTION OF THE RECOMBINANT HOXB4 PROTEIN CONTAINING
HIS TAG AT C-TERMINAL AND USE THEREOF

<130> P36142

<140> PCT/CN2006/000646
<141> 2006-04-11

<150> CN200610003108.1
<151> 2006-02-10

<160> 4

<170> Patent Version 3.3

<210> 1
<211> 25
<212> DNA
<213> Artificial Sequence

<220>
<223> Primer

<400> 1
ctccatggct atgagttctt ttttg                                          25

<210> 2
<211> 31
<212> DNA
<213> Artificial Sequence

<400> 2
<212> DNA
<213> Artificial Sequence

<220>
<223> Primer

<400> 2
atgatgatga tgatgatgat ggagcgcgcg g                                    31

<210> 3
<211> 27
<212> DNA
<213> Artificial Sequence

<220>
<223> Primer

<400> 3
cagaattcct aatgatgatg atgatga                                          27

<210> 4
<211> 1053
<212> DNA
<213> Artificial Sequence

<220>
<221>
<222> (1010)..(1024)
<223> C-terminal histidine tag coding sequence added into pTAT-HOXB4 construct

<400> 4

gtttcctcta gaataatttt gtttacttta agaaggagat atacatatgc ggggttctca       60

tcatcatcat catcatggta tggctagcat gactggtgga cagcaaatgg gtcgggatct      120

gtacgacgat gacgataagg atcgatgggg atcgatgggg atccaagctt ggctacggcc      180

ccgccagcgc cgccgcggtg gatccaccat gtccggctat ccatatgacg tcccagacta      240

tgctggctcc atggctatga gttctttttt gatcaactca aactatgtcg accccaagtt      300

ccctccatgc gaggaatatt cacagagcga ttacctaccc agcgaccact cgcccgggta      360

ctacgccggc ggccagaggc gagagagcag cttccagccg gaggcgggct tcgggcggcg      420

cgcggcgtgc accgtgcagc gctacgcggc ctgccgggac cctgggcccc cgccgcctcc      480

gccaccaccc ccgccgcccc cgccaccgcc cggtctgtcc cctcgggctc ctgcgccgcc      540

acccgccggg gccctcctcc cggagcccgg ccagcgctgc gaggcggtca gcagcagccc      600

cccgccgcct ccctgcgccc agaaccccct gcaccccagc ccgtcccact ccgcgtgcaa      660

agagcccgtc gtctacccct ggatgcgcaa agttcacgtg agcacggtaa accccaatta      720

cgccggcggg gagcccaagc gctctcggac cgcctacacg cgccagcagg tcttggagct      780

ggagaaggaa tttcactaca accgctacct gacacggcgc cggagggtgg agatcgccca      840

cgcgctctgc ctctccgagc gccagatcaa gatctggttc cagaaccggc gcatgaagtg      900

gaaaaaagac cacaagttgc ccaacaccaa gatccgctcg ggtggtgcgg caggctcagc      960

cggagggccc cctggccggc ccaatggagg cccccgcgcg ctccatcatc atcatcatca     1020

tcattaggaa ttcaaagctt gatccggctg cta 1053

## Description

### FIELD OF THE INVENTION

The present invention relates to a process for making the improved C-terminal Histidine tagged HOXB4 recombinant protein (TAT-HOXB4H) which containing at least 6 Histidine residues in the C-terminus. The present invention also relates to a DNA construct for expressing the TAT-HOXB4H recombinant protein in host cells, for which the increased productivity may facilitate stem cells expansion.

### BACKGROUND OF THE INVENTION

Umbilical cord blood (UCB) stem cells are naive cells which are often used to replace bone marrow transplantation. As bone marrow stem cells, UCB stem cells could divide and differentiate into millions of mature blood cells of all lineages. The relative ease of procurement and the lowerthan-anticipated risk of severe acute graft-versus-host disease (GVHD) has made UCB transplantation an appealing alternative to bone-marrow-derived hematopoietic stem cells. Because of the amount of UBC stem cells from single individual is limited, the cell number required for transplantation is only sufficient for children; more cell number would be required if the same procedure is applied to an adult.

To overcome this limitation, *in vitro* expansion of umbilical cord blood stem cell is necessary and has become the most challenging technique in clinical therapy. Recent evidences indicated that homeobox (HOX) transcription factors are involved in the regulation of normal and leukemic hematopoiesis. Among the HOX genes, HOXB4 is selectively expressed in primitive hematopoietic cells but is down-regulated in committed progenitors and more mature hematopoietic cells. Retroviral overexpression of HOXB4 enhances HSC self renewal and induces up to 1000 fold in vivo expansion in mouse bone marrow. In addition, the HOXB4 expanded HSCs retained their normal differentiation and long-term repopulation potential, and no hematologic abnormalities were detected in large groups of mice receiving transplants. (See, for example, Antonchuk, J., Sauvageau, G., and Humphries, R.K., Exp. Hematol. 29, 1125-1134, 2041; and Antonchuk, J., Sauvageau, G., and Humphries, R.K., Cell 109, 39-45, 2002).

Recent results showed that incubation of stromal cells genetically engineered to secrete HOXB4 or recombinant TAT-HOXB4 with either UCB or mouse bone marrow stem cells expanded the stem cell pools 2.5 or 6 fold, respectively. (See, for example, Amsellem, S. et al., Nature Medicine 9, 1423-1427, 2003 ; and Krosl, J. et al., Nature Medicine 9, 1428-1432 , 2003). Recombinant TAT-HOXB4 protein treatment may be useful in stem cell expansion for its simplicity and no involvement of stromal cells. However, recombinant TAT-HOXB4 proteins were previously used to expand mouse bone marrow stem cells only and have never been applied to human PB or UCB.

Hematopoietic stem cells (HSCs) are commonly used in clinical transplantation protocols to treat a wide variety of diseases. However, efficient transplantation requires substantial amount of HSCs from different sources and may require expansion. Effective expansion of HSCs remained a technical hurdle in the development of advanced cell therapies Human homeobox B4 (HOXB4) gene was recently demonstrated to effectively expand HSCs in a retroviral or recombinant protein form. Recombinant TAT-HOXB4 proteins were used to expand stem cells in the laboratory scale without the risk of retroviral insertion or co-culture with bone marrow stromal cells. However, recombinant TAT-HOXB4 proteins were never used to expand human peripheral blood (PB) or umbilical cord blood (UCB) stem cells in a clinical scale.

Therefore, the present invention modified the TAT-HOXB4 to contain six histidine residues in the C-terminal (designated TAT-HOXB4H) whose yield was 3-4 fold of the original TAT-HOXB4 after purification. Furthermore, the mass-produced and purified the recombinant TAT-HOXB4H proteins exhibit the potential to clinical scale and uses.

### SUMMARY OF THE INVENTION

In one aspect of the invention, a method for making a C-terminal histidine tagged HOXB4 recombinant protein (TAT-HOXB4H) containing at least 6 histidine residues in the C-terminus is provided. The method comprises the steps of: (a) prepartion of a DNA construct containing at least 6 histidine-coding sequence at the the C-terminus of the open reading frame (ORF) of HOXB4; (b) tranforming the DNA construct obtained in step (a) into an appropriate host cell for expression; and (c) isolation and purification of expressed TAT-HOXB4H recombinant protein from the host cell lysate.

In one embodiment, the additional 5 histidine residues are inrtoduced to the C-terminus of the open reading frame (ORF) of TAT-HOXB4 in the DNA construct by PCR method. In another embodiment, the DNA construct is plasmid pTAT-HOXB4H. In a specific embodiment, the host cell is *E*. *coli.*

In another aspect, the invention provides a DNA construct for improving the productivity of recombinant protein TAT-HOXB4H, which is characterized by the presence of 7 histidine tags at the C-terminus of the ORF of HOXB4. In one embodiment, the DNA construct is plasmid pTAT-HOXB4H.

Also provided is a culture medium for expanding stem cells, comprising 5-100 nM of TAT-HOXB4H recombinant protein produced by the method according to the invention, and conventional nutrients, cytokines and antibiotics for stem cell expansion.

### BRIEF DESCRIPTION OF THE FIGURE

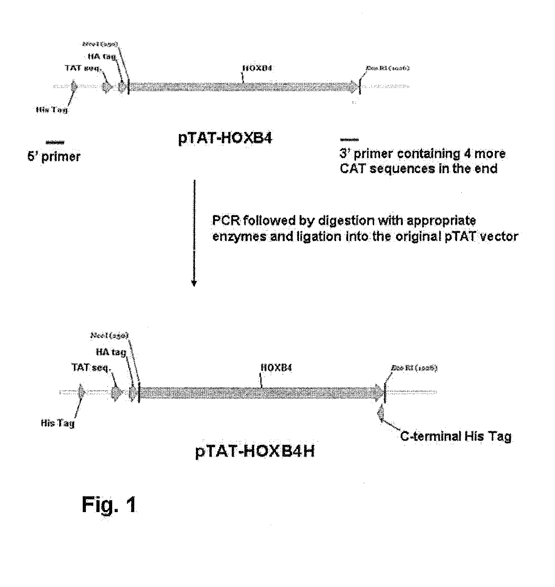

Fig 1. represents the construction of pTAT-HOXB4H which containing 7-Histidine tag at C-terminal.

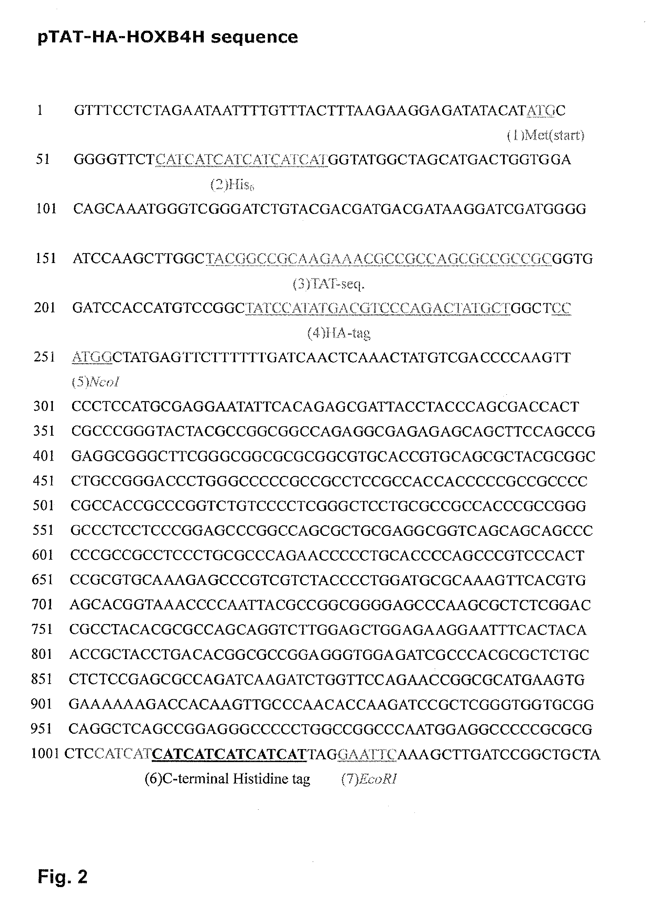

Fig 2. shows the DNA sequence of pTAT-HOXB4H. The additional histidine residues inrtoduced to the C-terminus in pTAT-HOXB4H are underlined as (6).
Fig 3. represents the final purified products from pTAT-HOXB4 and pTAT-HOXB4H expressions. The purification efficiency of pTAT-HOXB4H is 4-fold than that of pTAT-HOXB4.
Fig4. represents the expansion efficiency of pTAT-HOXB4 (hollow circle) and pTAT-HOXB4H (triangle) *in vitro.* A BSA (bovine serium albumin)-treated group is used as control (full circle).

### DETAILED DESCRIPTION OF THE INVENTION

The invention is further defined by reference to the following examples describing in detail the preparation and purification of TAT-HOXB4H of the invention. It will be apparent to those skilled in the art that many modifications, both to materials and methods, may be practiced without departing from the purpose and interest of this invention. The following examples are set forth to assist in understanding the invention and should not be construed as specifically limiting the invention described and claimed herein.

### Example 1. Construction of plasmid pTAT-HOXB4H

A DNA fragment containing the open reading frame (ORF) of HOXB4 and additional 4 histidine coding sequence was obtained by PCR amplification using plasmid pTAT-HOXB4 *(* Nature Medicine 9, 1428-1432 (2003)) as template, and following primer pair: 5'-ctccatggctatgagttcttttttg-3'and 5'-atgatgatgatgatgatggagcgcgcgg-3'. The amplified DNA fragment was then subjected to the PCR raction using following primer pair to abtained a new restriction site for EcoRI: 5'-ctccatggctatgagttcttttttg-3', and 5'-cagaattcctaatgatgatgatgatga-3'. The amplified DNA fragment was digested with restriction enzymes NcoI and EcoRI, and subcloned into the original backbone pTAT-HOXB4, which had been digested with the same two enzymes. After ligation, a novel plasmid pTAT-HOXB4H was obtained.

### Example 2. Purification and production of TAT-HOXB4 and TAT-HOXB4 recombinant proteins

The pTAT-HA-HOXB4 (or pTAT-HOXB4H) expression vector was transformed into *E. coli* strain B121(DE3)pLysS (Novagen). The transformed cell were grown overnight at 37°C. The overnight cultures were diluted into an initial OD₆₀₀ of 0.05. The cultures were grown to an OD₆₀₀ of 0.5 at 37°C, induced with 1 mM isopropyl-beta-D-thiogalactopyranoside (IPTG) at 37°C for 3 hr, with vigorous shaking. Following induction, cells were harvested by centrifugation and resuspended in Buffer A (8M Urea, 20mM HEPES, 0.5mM DTT and 100mM NaCl pH8.0). The cell suspension was passed three times through a French press, and the cell lysate cleared by centrifugation at 20,000 x g for 30 min. at 4°C, adjusted the supernatant to 10 mM Imidazole and loaded on the HisTrap chelating columns (Amersham Pharmacia). Bound proteins were eluted with 50, 100 and 250mM Imidazole in buffer A. HOXB4 containing fraction were loaded on a MonoSP column in Buffer B (4M urea, 20mM HEPES and 50mM NaCl pH6.5), eluted with 1.5M NaCl and 20mM HEPES (pH8.0) and desalted on PD-10 Sephadex G-25 column. HOXB4 protein was eluted from PD-10 Sephadex G-25 column with PBS in fractions with>99% purity, and all eluates were supplemented with 1% BSA and 10% glycerol, aliquoted and flash-frozen at -80 °C.

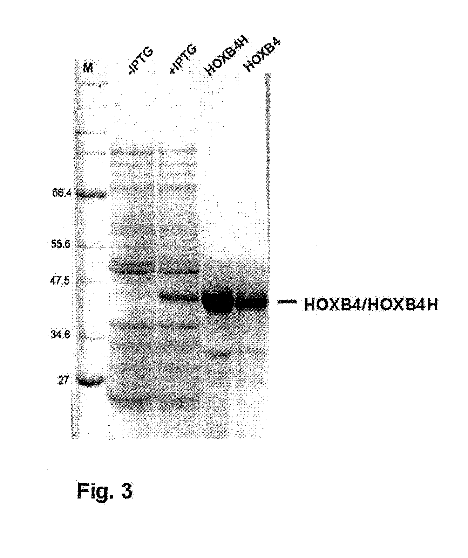

As the electrophoretic data showed in Fig.3, the recovery yield of recombinant TAT-HOXB4H produced according to the present invention was 3 to 5-flod higher than that from the original TAT-HOXB4 production.

The Bradford protein assay is one of several simple methods commonly used to determine the total protein concentration of a sample. The method is based on the proportional binding of the dye Coomassie to proteins. Within the linear range of the assay (~5-25 mcg/mL), the more protein present, the more Coomassie binds. Furthermore, the assay is colorimetric; as the protein concentration increases, the color of the test sample becomes darker. Coomassie absorbs at 595 nm. The protein concentration of a test sample is determined by comparison to that of a series of protein standards known to reproducibly exhibit a linear absorbance profile in this assay. The most widely used protein - Bovine Serum Albumin (BSA) was chosen as the standard in this test, although different protein standards can also be used.

By interpolation on the standard cure of OD₅₉₅ values vs indicated concentrations of BSA, total protein concentration in puried TAT-HOXB4H and TAT-HOXB4 protein sample was determined as 5.5 mg/L and 1.2 mg/L, respectively.

### Example 3. TAT-HOXB4 protein transduction and stem cell expansion

The red blood cells of human umbilical cord blood were removed by lysis at 4°C in 0.83% ammonium chloride with 0.1% sodiumbicarbonate (pH 7.0). The enriched white blood cell fraction was then used after being enriched for CD34+ cells using a high gradient magnetic cell separation procedure in which cells expression markers of mature human hematopoitic cells were removed on a StemSep column (StemCell tech.). The purified cell were cultured for 2 days in stem cell medium (DMEM with 10%FCS, 5ng/ml interleukin-3, 10ng/ml interleukin-6, 100ng/ml STF, penicillin 100 unit/ml and 100 ug/ml streptomycin), and then for 4 days in stem cell medium containing 15nM HOXB4 or 1%BSA. On day 3 (day 0 of treatment), 4x10⁵ cells/ml were resuspended in stem cell medium supplemented with BSA or HOXB4. Fresh BSA or HOXB4 proteins (50% initial protein amount in 5% of total culture medium) were then added every 3hr. After 12hr, FCS and cytokines were added to correct for the resulting 20% dilution of culture media. After 24h, cell were resuspended in fresh medium containing the protein of interest. On days 0, 2 and 4 of treatment, stem cells were counted with ISHAGE procedure (CD45/CD34 staining plus side and forward scatter evaluation).

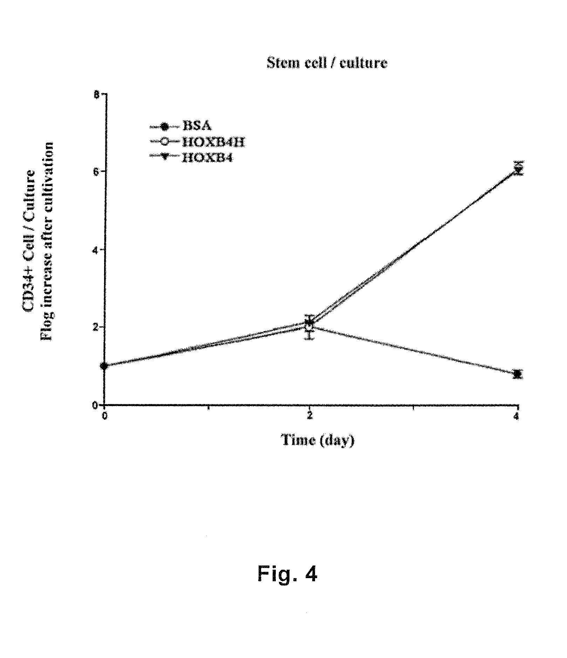

By the results represented in Fig. 4, the incubation of recombinant TAT-HOXB4H of the invention with UCB stem cells expanded the stem cell pools to about 6 fold, which similar to the efficiency of TAT-HOXB4 protein. It is indicated that recombinant TAT-HOXB4H, which caotains 4 additional histidine residues at the C-terminus, exhibits substantially the same benefits on stem cell proliferation as the original TAT-HOXB4 protein.

In the mice repopulating assay, cells were derived from 5000 human CD34⁺ initial cord blood cells at day 0, or from 4 days cocultures with TAT-HOXB4H protein. The cells were then collected and injected into irradiated (2.5 Gy) NOD-LtSz-scid/scid (NOD-SCID) mice, along with 5x10⁴ CD34⁻ irradiated accessory cells, on day 0. Seven weeks after transplantation, blood cells from transplanted animals were analyzed by flow cytometry for the presence of human CD45⁺ cells. Mice were considered to be positive for human UCB engraftment when at least 0.1 % CD45⁺ human cells were detected among mouse cells. Cells from positive mice were further phenotyped using the antibodies as follow, in which FITC-conjugated antibody to CD34 (Becton Dickinson) and phycoerythrin (PE)-conjugated antibody to CD45 (Becton Dickinson) were used for cell counting by ISHAGE. The presence of human cells in NOD-SCID mice was determined using human CD45-PE antibody, and immunophenotyping was done with mice Ly-5-FITC antibodies

As the result, the stem cells treated with the recombinant TAT-HOXB4H protein successfully incorporated into the bone marrow of mice, and about 0.05 to 0.07% CD45⁺ human leucocytes were detected in peripferal white blood cells, indicating that the expanded stem cells still possessed their pluripotency.

The provided results showed that TAT-HOXB4H expanded the HSCs from PB and UCB by 5-6 fold. The results from semisolid cloning assay, human long-term culture-initiating cells (LTC-ICs) assay and nonobese diabetic-severe combined immunodeficiency (NOD-SCID) mice repopulating assay all showed that TAT-HOXB4H expanded HSCs and retained the repopulating capacity and pluripotentiality. A new serum free medium supporting *in vitro* HSCs expansion was adapted to facilitate future clinical trial. Our practical approaches set the stage for embarking on the phase I clinical trial to treat different hematological malignancies, solid tumors and non-hematological diseases.

## Claims

1. A method for making a C-terminal histidine tagged HOXB4 recombinant protein (TAT-HOXB4H) containing at least 6 histidine residues in the C-terminus, which method comprising: preparing a DNA construct containing at least 6 histidine-coding sequence at the the C-terminus of the open reading frame (ORF) of HOXB4; tranforming the DNA construct into host cell for expression to express the TAT-HOXB4H recombinant protein; and isolation and purification of the expressed recombinant protein from host cell lysate.

2. The method for making a C-terminal histidine tagged HOXB4 recombinant protein containing at least 6 histidine residues in the C-terminus of claim 1, wherein the additional 5 histidine residues are inrtoduced to the C-terminus of the open reading frame of TAT-HOXB4 in the DNA construct by polymerase chain reaction method.

3. The method for making a C-terminal histidine tagged HOXB4 recombinant protein containing at least 6 histidine residues in the C-terminus of claim 1, which is **characterized** that the host cell is *E*. *coli.*

4. A DNA construct for improving the productivity of recombinant protein TAT-HOXB4H, which is **characterized by** the introduction of 7 histidine tags at the C-terminus of the ORF of TAT-HOXB4.

5. The DNA construct of claim 4, which is plasmid pTAT-HOXB4H.

6. A culture medium for expanding stem cells, comprising 5-100 nM of TAT-HOXB4H recombinant protein obtained by the method of claim 1, and appropriate nutrients, cytokines and antibiotics for stem cell expansion.

7. The culture medium of claim 6, which is **characterized** that the stem cells are umbilical cord blood (UCB) stem cells.

8. The culture medium of claim 6, which is **characterized** that the stem cells are hematopoietic stem cells.
